Europäisches Patentamt

European Patent Office

Office européen des brevets

(19)

(11) Veröffentlichungsnummer : **0 387 820 B1**

(12)
# EUROPÄISCHE PATENTSCHRIFT

(45) Veröffentlichungstag der Patentschrift :
**04.08.93 Patentblatt 93/31**

(51) Int. Cl.$^5$ : **C07C 46/08,** C07C 50/02

(21) Anmeldenummer : **90104775.3**

(22) Anmeldetag : **14.03.90**

(54) **Verfahren zur Herstellung von 2,3,5-Trimethyl-p-benzochinon.**

(30) Priorität : **17.03.89 DE 3908768**

(43) Veröffentlichungstag der Anmeldung :
**19.09.90 Patentblatt 90/38**

(45) Bekanntmachung des Hinweises auf die
Patenterteilung :
**04.08.93 Patentblatt 93/31**

(84) Benannte Vertragsstaaten :
**BE CH DE FR GB IT LI NL**

(56) Entgegenhaltungen :
**EP-A- 0 127 888**
**EP-A- 0 294 584**
**CHEMICAL ABSTRACTS, Band 104, 1986,**
**Seite 717, Spalte 2, Zusammenfassung Nr.**
**206913z, Columbus, Ohio, US; & JP-A-60 255**
**746**

(73) Patentinhaber : **BASF Aktiengesellschaft**
**Carl-Bosch-Strasse 38**
**W-6700 Ludwigshafen (DE)**

(72) Erfinder : **Hoercher, Ulrich, Dr.**
**Kolpingstrasse 15**
**W-6800 Mannheim 1 (DE)**
Erfinder : **Jessel, Barbara, Dr.**
**Hochfeldstrasse 26**
**W-6700 Ludwigshafen (DE)**
Erfinder : **Bockstiegel, Bernhard, Dr.**
**Chenoverstrasse 11**
**W-6703 Limburgerhof (DE)**
Erfinder : **Grafen, Paul, Dr.**
**Suedtiroler Ring 20**
**W-6719 Weisenheim (DE)**
Erfinder : **Laas, Harald, Dr.**
**Sohlstrasse 105**
**W-6701 Maxdorf (DE)**

EP 0 387 820 B1

## Beschreibung

Die vorliegende Erfindung betrifft ein verbessertes Verfahren zur Herstellung von 2,3,5-Trimethyl-p-benzochinon (im folgenden TMC genannt) durch Oxidation von 2,3,6-Trimethyl-phenol (im folgenden TMP genannt) mit gasförmigem Sauerstoff in Anwesenheit eines Kupferhalogenid enthaltenden Katalysators.

TMC ist ein wichtiges Zwischenprodukt für die Herstellung von Trimethylhydrochinon, einem essentiellen Vorprodukt für die Synthese von $\alpha$-Tocopherol (Vitamin E).

In Anbetracht seiner großen Bedeutung hat es nicht an Versuchen gefehlt, ein besonders vorteilhaftes Verfahren zur Herstellung von TMC zu finden.

So sind bereits zahlreiche Methoden für die Oxidation von TMP und anderen Phenolen zu den entsprechenden Benzochinonen mit Sauerstoff als Oxidationsmittel bekannt. Gemäß dem Verfahren des deutschen Patents 2 221 624 werden hierfür Kupfer-II-chlorid als Katalysator und Dimethylformamid (DMF), Ethylenglykolmonomethylether oder Aceton, gegebenenfalls in Kombination mit Wasser, als Lösungsmittel verwendet.

Die Oxidation von TMP zu TMC läßt sich auch anschließend an die Herstellung von TMP aus 2,3,6-Trimethyl-2-cyclohexen-1-on als Eintopfreaktion durchführen. Gemäß dem Verfahren der EP 93 880 werden hierfür niedere Alkohole (insbesondere Isopropanol) als Lösungsmittel verwendet, gemäß der EP 35 635 bevorzugt DMF, Acetonitril, Diethylenglycolmonomethylether oder Diethylenglyoldimethylether. Oxidiert wird bei beiden Verfahren mit Luft oder Sauerstoff unter Katalyse von Kupfer-II-halogeniden.

Gemäß dem Verfahren des deutschen Patents 2 827 552 wird für die Oxidation von Phenol zu p-Benzochinon Kupfer-I- oder Kupfer-II-chlorid, dem elementares Metall sowie ein Alkali- oder Erdalkalihalogenid zugesetzt wurde, als Katalysator verwendet. Als Lösungsmittel werden Methanol, Acetonitril oder DMF genannt. Gemäß den Verfahren von EP 70 665 und EP 107 427 werden Kupfer-II-halogenid mit einer Alkali- bzw. Erdalkalibase als Promotor als Katalysator und Acetonitril als Lösungsmittel verwendet.

In der DE 2 444 234 wird für die Oxidation von TMP die Anwendung eines Sauerstoffpartialdrucks bis zu 52 bar beschrieben, wobei dem Kupferkatalysator in DMF ein Thiocyanat, Cyanat oder Cyanid zugesetzt wird.

Aus EP-A 0 294 584 ist ein Verfahren zur Herstellung von TMC bekannt, bei dem TMP in Gegenwart einer wäßrigen Lösung von $CuCl_2$ und LiCl in einem Gemisch aus einem aromatischen Kohlenwasserstoff und einem niederen alphatischen Alkohol oxidiert wird.

Ein recht gutes Verfahren ist das gemäß EP 127 888. In diesem Patent wird ein Verfahren zur Herstellung von 2,3,5-Trimethyl-p-benzochinon durch Oxidieren von Trimethylphenol mit $O_2$ in einem aus Wasser und einem aliphatischen $C_4$- bis $C_{10}$-Alkohol bestehenden 2-phasigen Reaktionsmedium und in Gegenwart eines Kupfer-Alkalimetall-Halogen-Komplexes der allgemeinen Formel $M_1[Cu(II)_mX_n]_p$, in der M ein Alkalimetall oder Ammonium, X ein Halogenatom, 1 eine ganze Zahl von 1 bis 3, m und p die Zahlen 1 oder 2, und n eine ganze Zahl von 3 bis 6, wobei gilt 1 + 2 mp = n.p, beschrieben. Gemäß diesem Verfahren werden gute Ausbeuten an 2,3,5-Trimethyl-p-benzochinon erhalten. Aus EP 167 153 ist weiterhin ein Verfahren zur Herstellung von Trimethyl-p-benzochinon bekannt, welches mit dem vorher beschriebenen Verfahren im wesentlichen identisch ist, nur daß dem Katalysator zusätzlich Kupfer(I)hydroxid oder Kupfer(I)chlorid zugefügt wird und ein langsames Zutropfen der alkoholischen TMP-Lösung zu der wäßrigen Katalysatorlösung als wesentlich angegeben wird.

Ein Vorteil der 3 letzten genannten Verfahren ist die leichte Abtrennbarkeit des Katalysators vom Produkt durch das Arbeiten in einem 2-phasigen Lösungsmittelsystem. Ein Nachteil der beiden letztgenannten Verfahren ist, daß der als Katalysator verwendete Kupfer-Alkalimetall-Halogen-Komplex extra vorher hergestellt werden muß. Nachteilig an diesen beiden sowie den anderen bekannten Verfahren ist, daß brennbare Lösungsmittel verwendet werden. Üblicherweise vermeidet man die aus der Verwendung brennbarer Lösungsmittel resultierende Explosionsgefahr in technischen Anlagen, indem man den Prozeß unter Stickstoff durchführt. Das ist aber bei den beschriebenen Verfahren nicht möglich, da hier gasförmiger Sauerstoff oder Luft als Oxidationsmittel benötigt werden.

Für die Sicherheit bei der technischen Durchführung ist es daher wichtig, daß der Flammpunkt des verwendeten Lösungsmittels möglichst weit oberhalb der Reaktionstemperatur liegt, um die Möglichkeit des Auftretens von verheerenden Explosionen auszuschließen. Der Flammpunkt sollte so hoch liegen, daß auch bei Temperaturerhöhungen durch einen kurzzeitig unkontrollierten Verlauf der Reaktion oder bei technischen Störungen in der Anlage keine Explosionsgefahr zu erwarten ist. Dies ist bei den zitierten Verfahren nicht der Fall. In Tabelle 1 sind die Flammpunkte der in den zitierten Schriften bevorzugt verwendeten Lösungsmittel aufgeführt. Die zur Erzielung guter Ausbeuten erforderlichen bevorzugten Reaktionstemperaturen liegen in allen Fällen bei 60°C oder höher.

Tabelle 1

| Lösungsmittel | Flammpunkt in °C |
| --- | --- |
| DMF | 57 |
| Ethylenglykolmonomethylether | 46 |
| Aceton | -17 |
| Isopropanol | 22 |
| Diethylenglkyolmonomethylether | 83 |
| Acetonitril | 5 |
| Diethylenglykoldimethylether | 70 |
| Methanol | 11 |
| Hexanol | 60 |
| Heptanol | 73 |
| Oktanol | 81 |
| Nonanol | 75 |
| Dekanol | 82 |

Lediglich die $C_7$- bis $C_{10}$-Alkanole Heptanol, Oktanol, Nonanol und Dekanol, die gemäß den Verfahren von EP 167 153 und EP 127 888 als Lösungsmittel verwendet werden können, liegen mit ihren Flammpunkten geringfügig über den dort bevorzugten Reaktionstemperaturen von etwa 60°C. Für eine technische Anlage ist aber ein größerer Sicherheitsabstand wünschenswert, um die Explosionsgefahr möglichst gering zu halten.

Es war daher die Aufgabe der Erfindung, ein Verfahren zur katalytischen Oxidation von TMP zu TMC zu entwickeln, bei dem ein Lösungsmittel mit einem hohen Flammpunkt und einer möglichst großen Differenz zwischen der Reaktionstemperatur und dem Flammpunkt verwendet wird, bei dem gleichzeitig gute Ausbeuten an TMC erhalten werden, der Katalysator leicht herstellbar und leicht aus dem Reaktionsgemisch rückgewinnbar ist, und außerdem der Siedepunkt des Lösungsmittels möglichst weit vom Siedepunkt des TMC entfernt ist, so daß eine einfache destillative Abtrennung und Reinigung des TMC sowie eine einfache Rückgewinnung des Lösungsmittels gewährleistet sind.

Es wurde nun überraschend gefunden, daß sich die Aufgabe dieser Erfindung weitgehend lösen läßt, wenn man als Lösungsmittelsystem ein Gemisch aus Wasser und einem gesättigten aliphatischen Alkohol mit 12 bis 18, vorzugsweise 12 bis 14 C-Atomen einsetzt, insbesondere wenn als Katalysatoren Kupfer-II-halogenide verwendet werden, denen zur Aktivitätssteigerung Erdalkali- oder Alkalihalogenide zugesetzt werden.

Dieses Ergebnis war insofern nicht zu erwarten, als es sich bei den genannten Alkoholen, wie dem Dodekanol, Tetradekanol, Hexadekanol oder Oktadekanol um wachsartige Feststoffe mit hydrophoben Eigenschaften handelt, die eine wesentlich geringere Wasserlöslichkeit als die in EP 127 888 beschriebenen $C_5$- bis $C_{10}$-Alkohole besitzen. Daher waren schlechtere Wechselwirkungen der organischen Phase mit der wäßrigen Katalysatorlösung und somit eine geringe Eignung für die genannte 2-Phasenreaktion zu erwarten. Überraschenderweise zeigte sich jedoch, daß die Oxidation von TMP zu TMC unter den genannten Bedingungen sehr vorteilhaft verläuft, und dies bei Temperaturen, die ausreichend weit vom Flammpunkt des jeweiligen Lösungsmittels entfernt sind. In Tabelle 2 sind einige der erfindungsgemäß verwendeten Alkohole mit den in ihnen bevorzugten Reaktionstemperaturen und den Flammpunkten zusammengefaßt.

Tabelle 2

| Alkohol | Flammpunkt | Siedepunkt | Bevorzugte Reaktionstemperatur |
| --- | --- | --- | --- |
| Dodekanol | 127°C | 261°C | 80°C |
| Tetradekanol | 141°C | 289°C | 80 - 90°C |
| Hexadekanol | 135°C | 180°C/1.3 KPa (10 mm of Hg) | 80 - 90°C |
| Octadekanol | 192°C | 170°C/0.26 KPa (2 mm of Hg) | 80 - 90°C |

Aus Tabelle 2 geht hervor, daß in allen Fällen der Abstand zwischen Reaktionstemperatur und Flammpunkt deutlich über 40°C liegt, so daß Explosionen durch Zündfunken im Gasraum ausgeschlossen sind. Der Sicherheitsabstand ist deutlich höher als bei den in EP 127 888 und EP 167 153 beschriebenen $C_5$- bis $C_{10}$-Alkoholen. Damit wird eine gefahrlose technische Realisierung der katalytischen Oxidation von TMP zu TMC mit gasförmigem Sauerstoff möglich.

Ein weiterer Vorteil ist der große Abstand der Siedepunkte der verwendeten Alkohole (siehe Tabelle 2) vom Siedepunkt des TMC (198°C). Hierdurch wird eine einfache destillative Abtrennung von TMC aus dem Lösungsmittel sowie eine einfache Rückgewinnung des Alkohols möglich. Durch das Arbeiten in einem 2-Phasensystem ist außerdem der Vorteil einer einfachen Abtrennung und Rückführung der wäßrigen Katalysatorlösung gegeben.

Gegenstand der Erfindung ist daher ein Verfahren zur Herstellung von 2,3,5-Trimethyl-p-benzochinon durch Oxidation von Trimethylphenol mit Sauerstoff oder einem sauerstoffhaltigen Gas in Gegenwart eines Kupfer(II)halogenid enthaltenden Katalysators in einem zweiphasigen Reaktionsmedium bestehend aus Wasser und einem aliphatischen Alkohol bei erhöhter Temperatur, das dadurch gekennzeichnet ist, daß man die Umsetzung in einem Gemisch aus Wasser und einem gesättigten aliphatischen Alkohol mit 12 bis 18 C-Atomen und einem Flammpunkt oberhalb von 120°C und bei Temperaturen zwischen 60 und 100°C durchführt.

Als gesättigte aliphatische Alkohole mit 12 bis 18 C-Atomen mit einem Flammpunkt oberhalb 120°C seien beispielsweise 1-Dodekanol, 1-Tetradekanol, 1-Hexadekanol oder 1-Octadekanol, insbesondere 1-Dodekanol genannt.

Gute Ausbeuten an TMC werden erhalten, wenn man die erfindungsgemäße Umsetzung in Gegenwart von einem Kupfer(II)halogenid und einem Erdalkalichlorid oder einem Alkalichlorid als Katalysatorsystem durchführt.

Als geeignete Kupfer(II)halogenide seien insbesondere $CuCl_2$ und $CuBr_2$ genannt.

Bei den zur Aktivitätssteigerung zugesetzten Erdalkalihalogeniden handelt es sich im wesentlichen um $MgCl_2$, $MgBr_2$, $CaCl_2$ und $CaBr_2$, insbesondere $MgCl_2$ und $CaCl_2$.

Als aktivitätssteigernde Alkalihalogenide seien vor allem LiCl, LiBr, NaCl und NaBr, insbesondere LiCl und NaCl genannt.

Die beiden Komponenten des Katalysatorsystems, das Kupfer(II)halogenid und das Erdalkali- bzw. Alkalihalogenid werden dem Reaktionsgemisch in Form ihrer wäßrigen Lösung zugefügt oder aber beide in der wäßrigen Phase des Reaktionsgemisches gelöst. Die wäßrige Katalysator enthaltende Lösung ist also auf sehr einfache Weise herstellbar. Das aufwendige Vorfertigen eines Komplexkatalysators, wie es gemäß EP 127 888 und EP 167 153 beschrieben wird, ist nicht notwendig.

Die Konzentration des Kupferhalogenids in Wasser kann 5 bis 70 Gew.%, vorzugsweise 10 bis 50 Gew.%, betragen. Die Alkali- oder Erdalkalihalogenide werden bevorzugt in Konzentrationen von 5 bis 80 Gew.% eingesetzt.

Die Menge des Kupfer-II-halogenids in Mol beträgt das 0,1- bis 10-fache, vorzugsweise das 0,4- bis 2-fache der eingesetzten molaren TMP-Menge. Die Alkali- oder Erdalkalihalogenide werden in der 0,1- bis 10-fachen, vorzugsweise 0,5- bis 5-fachen molaren Menge bezogen auf TMP eingesetzt.

Zusätzlich zu den Alkali- oder Erdalkalihalogeniden können auch noch andere aus dem Stand der Technik bekannte Aktivatoren, vor allem Kupfer-I-Salze, eingesetzt werden.

Die Umsetzung kann diskontinuierlich oder kontinuierlich durchgeführt werden.

Zur Durchführung der Reaktion wird das TMP in der Alkoholkomponente des Lösungsmittelsystems gelöst. Dafür muß der Alkohol aufgeschmolzen werden. Je nach Schmelzpunkt des verwendeten Alkohols und der Schmelzpunkterniedrigung, die durch das Lösen von TMP hervorgerufen wird, sind die TMP-Lösungen bei Raumtemperatur fest oder flüssig und müssen gegebenenfalls bei höheren Temperaturen gehandhabt werden. Die Konzentration von TMP in der Alkohollösung liegt im allgemeinen zwischen 5 und 80, vorzugsweise zwischen 10 und 50 Gew.%. Das Mengenverhältnis von wäßriger Phase zu Alkoholphase kann im Bereich von 10:1 bis 1:10 schwanken, bevorzugt wird der Bereich von 3:1 bis 1:3.

Als Oxidationsmittel kann reiner Sauerstoff oder ein sauerstoffhaltiges Gas, wie Luft eingesetzt werden.

Für die Zugabe der alkoholischen TMP-Lösung zur wäßrigen Katalysatorlösung gibt es 2 Möglichkeiten. Entweder wird die Gesamtmenge sofort zu Beginn der Reaktion zugefügt oder aber während der Reaktion langsam zugetropft.

Die Reaktionstemperatur ist prinzipiell nicht auf einen engen Bereich limitiert. Sie hängt davon ab, welche Anforderungen man unter dem Aspekt der Anlagensicherheit an den Abstand zum Flammpunkt des jeweiligen Alkohols stellt. Jedoch ist es ein besonderer Vorzug dieser Erfindung, daß der Abstand zwischen Reaktionstemperatur und dem Flammpunkt des verwendeten Lösungsmittels mit über 40°C erheblich höher gewählt werden kann als in bisher bekannten Verfahren. Beispiele für bevorzugte Temperaturen enthält Tabelle 2. Als Lösungsmittel ist prinzipiell jeder gesättigte, aliphatische Alkohol geeignet, der aufgrund seines Molgewichts einen ausreichend hohen Siedepunkt und Flammpunkt besitzt. Bevorzugt werden die in Tabelle 2 aufgeführten Alkohole, die großtechnisch aus pflanzlichen Fettsäuren hergestellt werden und daher in großen Mengen verfügbar sind.

Die folgenden Beispiele sollen die Erfindung verdeutlichen, ohne sie einzuengen.

Beispiele 1 bis 8

In einem 500-ml-Glaskolben wurde jeweils eine Lösung der aus Tabelle 3 ersichtlichen Mengen an Kupfer(II)halogenid und Alkali- bzw. Erdalkalihalogenid in 100 g Wasser vorgelegt, die Lösung mit einem Wasserbad auf die in Tabelle 3 angegebene Temperatur erwärmt und der Gasraum der Apparatur 2mal evakuiert und mit Sauerstoff belüftet. Anschließend wurde eine Lösung von jeweils 34 g TMP (0.25 Mol) in 100 g des in Tabelle 3 angegebenen Alkohols entweder sofort vollständig hinzugefügt oder im Verlauf von 3 Stunden (h) zur Katalysatorlösung hinzugetropft. Dabei wurde unter Einhaltung der angegebenen Temperatur das Reaktionsgemisch mit einem Magnetrührer bei 1100 U/min gerührt und aus einer Gasbürette Sauerstoff in den Gasraum des Glaskolbens geleitet, so daß der Verlauf der Reaktion am Sauerstoffverbrauch verfolgt werden konnte. Nach Beendigung der Sauerstoffaufnahme wurde die organische Phase von der wäßrigen Phase abgetrennt. Die organische Phase wurde 2mal bei 60 bis 70°C mit Wasser gewaschen und die Ausbeute durch Gaschromatographie mit internem Standard ermittelt. Der TMP-Umsatz lag in allen Fällen bei 100 %.

Tabelle 3

| Beispiel Nr. | Alkohol | Reaktions- temperatur [°C] | Katalysator (Molmenge) | TMP/Alkohol- Zugabezeit [Min] | $O_2$-Aufnahme- Zeit [Min] | TMC-Ausbeute [% der Theorie] |
|---|---|---|---|---|---|---|
| 1 | 1-Dodekanol | 80 | $CuCl_2$ (0.25) $MgCl_2$ (0.125) | 180 | 220 | 94.1 |
| 2 | 1-Dodekanol | 80 | $CuCl_2$ (0.25) $MgCl_2$ (0.25) | 180 | 210 | 94.9 |
| 3 | 1-Dodekanol | 80 | $CuCl_2$ (0.25) $CaCl_2$ (0.25) | 180 | 210 | 84.9 |
| 4 | 1-Dodekanol | 80 | $CuCl_2$ (0.25) $CaCl_2$ (0.25) | sofort | 70 | 82 |
| 5 | 1-Dodekanol | 80 | $CuCl_2$ (0.25) LiCl (0.50) | 180 | 210 | 93.4 |
| 6 | 1-Dodekanol | 80 | $CuCl_2$ (0.25) LiCl (0.50) | sofort | 60 | 81.1 |
| 7 | 1-Hexadekanol | 90 | $CuCl_2$ (0.25) LiCl (0.50) | sofort | 80 | 83.9 |
| 8 | 1-Oktadekanol | 90 | $CuCl_2$ (0.25) LiCl (0.50) | sofort | 90 | 83.4 |
| 9 | 1-Hexadekanol | 80 | $CuCl_2$ (0.25) $MgCl_2$ (0.25) | 180 | 220 | 92 |
| 10 | 1-Dodekanol | 80 | $CuCl_2$ (0.25) LiBr (0.25) | 180 | 210 | 94 |

EP 0 387 820 B1

**Patentansprüche**

1. Verfahren zur Herstellung von 2,3,5-Trimethyl-p-benzochinon durch Oxidation von Trimethylphenol mit Sauerstoff oder einem sauerstoffhaltigen Gas in Gegenwart eines Kupfer(II)halogenid enthaltenden Katalysators in einem zweiphasigen Reaktionsmedium bestehend aus Wasser und einem aliphatischen Alkohol bei erhöhter Temperatur, dadurch gekennzeichnet, daß man die Umsetzung in einem Gemisch aus Wasser und einem gesättigten aliphatischen Alkohol mit 12 bis 18 C-Atomen und einem Flammpunkt oberhalb von 120°C und bei Temperaturen zwischen 60 und 100°C durchführt.

2. Verfahren zur Herstellung von 2,3,5-Trimethyl-p-benzochinon gemäß Anspruch 1, dadurch gekennzeichnet, daß man die Umsetzung in einem Gemisch aus Wasser und 1-Dodekanol, 1-Tetradekanol, 1-Hexadekanol oder 1-Oktadekanol durchführt.

3. Verfahren zur Herstellung von 2,3,5-Trimethyl-p-benzochinon gemäß Anspruch 1, dadurch gekennzeichnet, daß man die Umsetzung in einem Gemisch aus Wasser und 1-Dodekanol durchführt.

4. Verfahren zur Herstellung von 2,3,5-Trimethyl-p-benzochinon gemäß Anspruch 1, dadurch gekennzeichnet, daß man die Umsetzung in Gegenwart von einem Kupfer(II)halogenid und einem Erdalkalihalogenid als Katalysatorsystem durchführt.

5. Verfahren zur Herstellung von 2,3,5-Trimethyl-p-benzochinon gemäß Anspruch 4, dadurch gekennzeichnet, daß man als Erdalkalihalogenid Magnesiumchlorid oder Calciumchlorid verwendet.

6. Verfahren zur Herstellung von 2,3,5-Trimethyl-p-benzochinon gemäß Anspruch 1, dadurch gekennzeichnet, daß man die Umsetzung in Gegenwart von einem Kupfer(II)halogenid und einem Alkalihalogenid als Katalysatorsystem durchführt.

7. Verfahren zur Herstellung von 2,3,5-Trimethyl-p-benzochinon gemäß Anspruch 6, dadurch gekennzeichnet, daß man als Alkalihalogenid Lithiumchlorid oder Natriumchlorid verwendet.

8. Verfahren zur Herstellung von 2,3,5-Trimethyl-p-benzochinon gemäß Anspruch 1, dadurch gekennzeichnet, daß man die alkoholische Lösung von Trimethylphenol während der Reaktion langsam in die wäßrige Katalysatorlösung eintropfen läßt.

**Claims**

1. A process for preparing 2,3,5-trimethyl-p-benzoquinone by oxidizing trimethylphenol with oxygen or an oxygen-containing gas in the presence of a catalyst containing copper(II) halide in a two-phase reaction medium composed of water and an aliphatic alcohol at elevated temperature, which comprises carrying out the reaction in a mixture of water and a saturated aliphatic alcohol with 12 to 18 C atoms and a flashpoint above 120°C and at from 60 to 100°C.

2. A process for preparing 2,3,5-trimethyl-p-benzoquinone as claimed in claim 1, wherein the reaction is carried out in a mixture of water and 1-dodecanol, 1-tetradecanol, 1-hexadecanol or 1-octadecanol.

3. A process for preparing 2,3,5-trimethyl-p-benzoquinone as claimed in claim 1, wherein the reaction is carried out in a mixture of water and 1-dodecanol.

4. A process for preparing 2,3,5-trimethyl-p-benzoquinone as claimed in claim 1, wherein the reaction is carried out in the presence of a copper(II) halide and of an alkaline earth metal halide as catalyst system.

5. A process for preparing 2,3,5-trimethyl-p-benzoquinone as claimed in claim 4, wherein magnesium chloride or calcium chloride is used as alkaline earth metal halide.

6. A process for preparing 2,3,5-trimethyl-p-benzoquinone as claimed in claim 1, wherein the reaction is carried out in the presence of a copper(II) halide and of an alkali metal halide as catalyst system.

7. A process for preparing 2,3,5-trimethyl-p-benzoquinone as claimed in claim 6, wherein lithium chloride or sodium chloride is used as alkali metal halide.

8. A process for preparing 2,3,5-trimethyl-p-benzoquinone as claimed in claim 1, wherein the alcoholic solution of trimethylphenol is slowly added dropwise during the reaction to the aqueous catalyst solution.

## Revendications

1. Procédé de préparation de 2,3,5-triméthyl-p-benzoquinone par oxydation de triméthylphénol avec de l'oxygène ou un gaz contenant de l'oxygène, en présence d'un catalyseur contenant un halogénure de cuivre (II), dans un milieu réactionnel à deux phases composé d'eau et d'un alcool aliphatique, à température élevée, caractérisé en ce qu'on conduit la réaction dans un mélange d'eau et d'un alcool aliphatique saturé à 12-18 atomes de carbone, ayant un point d'inflammation supérieur à 120°C, et à des températures comprises entre 60 et 100°C.

2. Procédé de préparation de 2,3,5-triméthyl-p-benzoquinone selon la revendication 1, caractérisé en ce qu'on conduit la réaction dans un mélange d'eau et de 1-dodécanol, de 1-tétradécanol, de 1-hexadécanol ou de 1-octadécanol.

3. Procédé de préparation de 2,3,5-triméthyl-p-benzoquinone selon la revendication 1, caractérisé en ce qu'on conduit la réaction dans un mélange d'eau et de 1-dodécanol.

4. Procédé de préparation de 2,3,5-triméthyl-p-benzoquinone selon la revendication 1, caractérisé en ce qu'on conduit la réaction en presence d'un halogénure de cuivre (II) et d'un halogenure de métal alcalino-terreux en tant que système catalytique.

5. Procédé de préparation de 2,3,5-triméthyl-p-benzoquinone selon la revendication 4, caractérisé en ce qu'on utilise, comme halogénure de métal alcalino-terreux, du chlorure de magnésium ou du chlorure de calcium.

6. Procédé de préparation de 2,3,5-triméthyl-p-benzoquinone selon la revendication 1, caractérisé en ce qu'on conduit la réaction en présence d'un halogénure de cuivre (II) et d'un halogénure de métal alcalin en tant que système catalytique.

7. Procédé de préparation de 2,3,5-triméthyl-p-benzoquinone selon la revendication 6, caractérisé en ce qu'on utilise, comme halogénure de métal alcalin, du chlorure de lithium ou du chlorure de sodium.

8. Procédé de préparation de 2,3,5-triméthyl-p-benzoquinone selon la revendication 1, caractérisé en ce qu'au cours de la réaction, on introduit goutte à goutte et lentement la solution alcoolique de triméthylphénol dans la solution aqueuse de catalyseur.